# EUROPEAN PATENT APPLICATION

(11) **EP 4 825 843 A1**
(43) Date of publication of application: **21.10.2026**
(21) Application number: 24902963.8
(22) Date of filing: 13.12.2024
(51) Int. Cl.: C07D 233/61, A61K 9/127, A61K 47/18

(54) **IMIDAZOLE LIPID AND USE THEREOF**

(30) Priority: 13.12.2023 CN 202311718923; 06.11.2024 CN 202411581341
(71) Applicant: Shanghai Regenelead Therapies Co., Ltd., Shanghai 201206 (CN)
(72) Inventor: LIU, Chongyi, Shanghai 201206 (CN); JIANG, Jun, Shanghai 201206 (CN); HUANG, Jian, Shanghai 201206 (CN); ZHU, Lingjian, Shanghai 201206 (CN); KUANG, Jingwen, Shanghai 201206 (CN); HONG, Min, Shanghai 201206 (CN); NING, Wei, Shanghai 201206 (CN); LIAO, Cheng, Shanghai 201206 (CN)
(74) Representative: Regimbeau
(86) International application number: PCT/CN2024/139179
(87) International publication number: WO 2025/124548

(57) **Abstract**

The present disclosure relates to an imidazole lipid and a use thereof. Specifically, provided are a compound represented by formula I or a salt thereof, and a lipid particle or a pharmaceutical composition containing the compound. The compound can be used as a delivery system, and is used for preparing drugs for preventing and/or treating cancers, infectious diseases, autoimmune diseases, neurodegenerative diseases, inflammation, etc. The definition of each group in formula (I) is as described in the description.

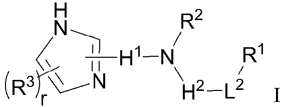

## Description

### TECHNICAL FIELD

The present disclosure pertains to the field of pharmaceuticals and relates to an imidazole lipid and use thereof.

### BACKGROUND

Gene therapy involves introducing exogenous normal genes into target cells to correct or compensate for diseases caused by defective and abnormal genes, achieving therapeutic purposes. Since the COVID-19 pandemic, gene therapy has become well-known to the public and has emerged as another hot topic following immunotherapy for cancer. It is gradually becoming a reality.

However, naked therapeutic genes are easily degraded by nucleases, exhibiting poor cellular uptake efficiency. Therefore, the preparation of a safe and efficient gene vector becomes a necessary condition for successful gene therapy. Mainstream nucleic acid drug delivery systems are divided into viral vector systems and non-viral systems. Lipid nanoparticle-mediated nucleic acid drug delivery is a primary method in non-viral delivery systems.

Nanoparticles consisting of cationic lipids and other helper lipids, such as lipid nanoparticles formed from cholesterol, phospholipids, and PEGylated lipids, can encapsulate nucleic acids to protect them from being degraded while promoting cellular uptake and reducing immune responses. In addition, when used to deliver bioactive ingredients into cells, lipid nanoparticles have other advantages, such as good targeting capability, minimal side effects, good stability, and relatively high transfection efficiency.

However, lipid nanoparticles are generally not highly selective for specific organs. For example, most lipid nanoparticles accumulate through biological processes in the liver, resulting in a reduction in the efficacy of active substances delivered into target organs. Therefore, there remains a need to develop new lipid nanoparticles that enable preferential delivery to specific organs and specific cells, such as better delivery systems that enhance *in vivo* T-cell engineering.

### SUMMARY

The present disclosure provides a compound represented by formula I or a salt thereof, 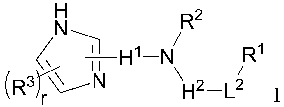 wherein:
H¹ is selected from the group consisting of C₁₋₆ alkylene, C₂₋₆ alkenylene, and C₁₋₆ heteroalkylene;
H² is selected from the group consisting of C₁₋₁₂ heteroalkylene;
R¹ is selected from the group consisting of C₁₋₂₄ alkyl, C₁₋₂₄ heteroalkyl, C₂₋₂₄ alkenyl, and C₂₋₂₄ heteroalkenyl;
R² is selected from the group consisting of C₁₋₂₄ alkyl, C₂₋₂₄ alkenyl, and -R^{b}-L¹-R^{c};
L¹ and L² are each independently selected from the group consisting of -C(O)O-, -OC(O)-, -C(O)-, -OC(O)O-, -O-, -S(O)_{q}-, -S-S-, -C(O)S-, -SC(O)-, -NR^{a}C(O)-, -C(O)NR^{a}-, -NR^{a}C(O)NR^{a}-, -NR^{a}C(O)O-, -OC(O)NR^{a}-, -P(O)(OR^{a})-, and a bond, wherein R^{a} is selected from the group consisting of hydrogen and C₁₋₆ alkyl, and q is selected from the group consisting of 0, 1, and 2;
R^{b} is selected from the group consisting of C₁₋₁₂ alkylene, C₁₋₁₂ heteroalkylene, C₁₋₁₂ alkenylene, and C₁₋₁₂ heteroalkenylene;
R^{c} is selected from the group consisting of C₁₋₂₄ alkyl, C₁₋₂₄ heteroalkyl, C₂₋₂₄ alkenyl, and C₂₋₂₄ heteroalkenyl;
each R³ is independently selected from the group consisting of halogen, amino, hydroxy, C₁₋₆ alkyl, C₁₋₆ alkylamino, C₁₋₆ alkoxy, C₃₋₇ cycloalkyl, and C₃₋₇ heterocycloalkyl;
r is selected from the group consisting of integers between 0 and 3, such as 1 or 2.

In some embodiments, in the compound represented by formula I or the salt thereof, H¹ is selected from the group consisting of C₁₋₆ alkylene, C₁₋₆ alkenylene, and C₁₋₆ heteroalkylene;
H² is selected from the group consisting of C₁₋₁₂ heteroalkylene;
R¹ is selected from the group consisting of C₁₋₂₄ alkyl, C₁₋₂₄ heteroalkyl, C₂₋₂₄ alkenyl, and C₂₋₂₄ heteroalkenyl;
R² is selected from the group consisting of C₁₋₂₄ alkyl, C₂₋₂₄ alkenyl, and -R^{b}-L¹-R^{c};
L¹ and L² are each independently selected from the group consisting of -C(O)O-, -OC(O)-, -C(O)-, -OC(O)O-, -O-, -S(O)_{q}-, -S-S-, -C(O)S-, -SC(O)-, -NR^{a}C(O)-, -C(O)NR^{a}-, -NR^{a}C(O)NR^{a}-, -NR^{a}C(O)O-, -OC(O)NR^{a}-, -P(O)(OR^{a})-, and a bond, wherein R^{a} is selected from the group consisting of hydrogen and C₁₋₆ alkyl, and q is selected from the group consisting of 0, 1, and 2;
R^{b} is selected from the group consisting of C₁₋₁₂ alkylene;
R^{c} is selected from the group consisting of C₁₋₂₄ alkyl, C₁₋₂₄ heteroalkyl, C₂₋₂₄ alkenyl, and C₂₋₂₄ heteroalkenyl;
each R³ is independently selected from the group consisting of halogen, amino, hydroxy, C₁₋₆ alkyl, C₁₋₆ alkylamino, C₁₋₆ alkoxy, C₃₋₇ cycloalkyl, and C₃₋₇ heterocycloalkyl;
r is selected from the group consisting of integers between 0 and 3, such as 1 or 2.

In some embodiments, in the compound represented by formula I or the salt thereof, H² is selected from the group consisting of C₁₋₁₂ heteroalkylene, wherein the heteroalkylene comprises at least one heteroatom selected from the group consisting of O, N, and S.

In some embodiments, in the compound represented by formula I or the salt thereof, H² is selected from the group consisting of C₁₋₁₂ heteroalkylene, such as C₂₋₉ heteroalkylene, C₂₋₇ heteroalkylene, C₂₋₄ heteroalkylene, C₃₋₅ heteroalkylene, or C₃₋₆ heteroalkylene.

In some embodiments, in the compound represented by formula I or the salt thereof, the heteroalkylene is heteroalkylene comprising at least one oxygen atom. In some embodiments, in the compound represented by formula I or the salt thereof, the heteroalkylene is heteroalkylene comprising two oxygen atoms. In some embodiments, in the compound represented by formula I or the salt thereof, the heteroalkylene is heteroalkylene comprising at least one nitrogen atom. In some embodiments, in the compound represented by formula I or the salt thereof, the heteroalkylene is heteroalkylene comprising at least one oxygen atom.

In some embodiments, in the compound represented by formula I or the salt thereof, R¹ is selected from the group consisting of C₁₋₂₄ alkyl, including C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, or C₂₃ alkyl.

In some other embodiments, in the compound represented by formula I or the salt thereof, R¹ is selected from the group consisting of C₄-₁₈ alkyl.

In some embodiments, in the compound represented by formula I or the salt thereof, R¹ is linear C₄-₁₈ alkyl.

In some embodiments, in the compound represented by formula I or the salt thereof, R¹ is branched C₄-₁₈ alkyl.

In some embodiments, in the compound represented by formula I or the salt thereof, R¹ is branched C₁₇ alkyl.

In some other embodiments, in the compound represented by formula I or the salt thereof, R¹ is selected from the group consisting of C₂₋₂₄ alkenyl, including C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, C₂₁ alkenyl, C₂₂ alkenyl, C₂₃ alkenyl, or C₂₄ alkenyl.

In the compound represented by formula I or the salt thereof provided in some other embodiments, R¹ is C₄-₁₈ alkenyl.

In some embodiments, in the compound represented by formula I or the salt thereof, R¹ is linear C₄-₁₈ alkenyl.

In some embodiments, in the compound represented by formula I or the salt thereof, R¹ is branched C₄-₁₈ alkenyl.

In the compound represented by formula I or the salt thereof provided in some other embodiments, R² is -R^{b}-L²-R^{c}, wherein R^{b}, L², or R^{c} is as defined above.

Further, the compound represented by formula I provided in some embodiments is a compound represented by formula II: 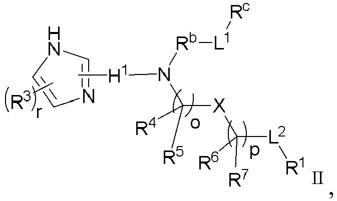 wherein X is -N(R^{d})- or -O-; o is selected from the group consisting of integers between 1 and 11, p is selected from the group consisting of integers between 1 and 5, and o + p is not greater than 12; R^{d} is selected from the group consisting of hydrogen and C₁₋₆ alkyl; R⁴, R⁵, R⁶, and R⁷ are each independently selected from the group consisting of hydrogen, halogen, hydroxy, C₁₋₆ alkyl, and C₁₋₆ alkoxy; L¹, L², H¹, R¹, R³, R^{b}, and R^{c} are as defined in the compound represented by formula I.

In some embodiments, in the compound represented by formula I or II or the salt thereof, R^{b} is selected from the group consisting of C₁₋₁₂ alkylene, including C₂ alkylene, C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula I or II or the salt thereof, R^{b} is linear C₁₋₁₂ alkylene.

In some embodiments, in the compound represented by formula I or II or the salt thereof, R^{b} is branched C₁₋₁₂ alkylene.

In some embodiments, in the compound represented by formula I or II or the salt thereof, R^{b} is linear C₇ alkylene.

In some embodiments, in the compound represented by formula I or II or the salt thereof, R^{b} is linear C₈ alkylene.

In some embodiments, in the compound represented by formula I or II or the salt thereof, R^{b} is linear C₉ alkylene.

In some other embodiments, in the compound represented by formula I or II or the salt thereof, R^{b} is selected from the group consisting of C₂₋₁₂ alkenylene, including C₃ alkenylene, C₄ alkenylene, C₅ alkenylene, C₆ alkenylene, C₇ alkenylene, C₈ alkenylene, C₉ alkenylene, C₁₀ alkenylene, C₁₁ alkenylene, or C₁₂ alkenylene.

In some embodiments, in the compound represented by formula I or II or the salt thereof, R^{b} is branched C₂₋₁₄ alkenylene.

In some embodiments, in the compound represented by formula I or II or the salt thereof, R^{b} is linear C₇ alkenylene.

In some embodiments, in the compound represented by formula I or II or the salt thereof, R^{b} is linear C₈ alkenylene.

In some embodiments, in the compound represented by formula I or II or the salt thereof, R^{b} is linear C₉ alkenylene.

In some other embodiments, in the compound represented by formula I or II or the salt thereof, R^{c} is selected from the group consisting of C₁₋₂₄ alkyl, including C₂ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, C₂₃ alkyl, or C₂₄ alkyl.

In some embodiments, in the compound represented by formula I or II or the salt thereof, R^{c} is linear C₁₋₁₂ alkyl.

In some embodiments, in the compound represented by formula I or II or the salt thereof, R^{c} is branched C₁₋₁₂ alkyl.

In some embodiments, in the compound represented by formula I or II or the salt thereof, R^{c} is branched C₁₁ alkyl.

In some embodiments, in the compound represented by formula I or II or the salt thereof, R^{c} is branched C₁₇ alkyl.

In some embodiments, in the compound represented by formula I or II or the salt thereof, R^{c} is linear C₉ alkyl.

In some embodiments, in the compound represented by formula I or II or the salt thereof, R^{c} is branched C₁₁₋₁₈ alkyl.

In some other embodiments, in the compound represented by formula I or II or the salt thereof, R^{c} is selected from the group consisting of C₂₋₂₄ alkenyl, including C₂ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, C₂₁ alkenyl, C₂₂ alkenyl, C₂₃ alkenyl, or C₂₄ alkenyl.

In some embodiments, in the compound represented by formula I or II or the salt thereof, R^{c} is linear C₂₋₁₂ alkenyl.

In some embodiments, in the compound represented by formula I or II or the salt thereof, R^{c} is branched C₂₋₁₂ alkenyl.

In some embodiments, in the compound represented by formula I or II or the salt thereof, R^{c} is branched C₁₁ alkenyl.

In some embodiments, in the compound represented by formula I or II or the salt thereof, R^{c} is branched C₁₇ alkenyl.

In some embodiments, in the compound represented by formula I or II or the salt thereof, R^{c} is linear C₉ alkenyl.

In some embodiments, in the compound represented by formula I or II or the salt thereof, p is selected from the group consisting of 1 and 2.

In some embodiments, in the compound represented by formula I or II or the salt thereof, p is selected from the group consisting of 3 and 4.

In some embodiments, in the compound represented by formula I or II or the salt thereof, o is selected from the group consisting of integers between 4 and 7, such as 5 or 6.

In some embodiments, in the compound represented by formula I or II or the salt thereof, X is -O-.

In some embodiments, in the compound represented by formula I or II or the salt thereof, p is selected from the group consisting of 1 and 2, and X is -O-. In some embodiments, in the compound represented by formula I or II or the salt thereof, p is selected from the group consisting of 3 and 4, o is selected from the group consisting of 5 and 6, and X is -O-.

In some embodiments, in the compound represented by formula I or II or the salt thereof, R⁴ and R⁵ are each independently selected from the group consisting of C₁₋₆ alkyl, such as methyl, ethyl, or propyl.

In some embodiments, in the compound represented by formula I or II or the salt thereof, R⁶ and R⁷ are each independently selected from the group consisting of C₁₋₆ alkyl, such as methyl, ethyl, or propyl.

In some embodiments, in the compound represented by formula I or II or the salt thereof, R⁴, R⁵, R⁶, and R⁷ are each independently selected from the group consisting of hydrogen.

In some embodiments, in the compound represented by formula I or II or the salt thereof, p is selected from the group consisting of 3 and 4, and o is selected from the group consisting of 5 and 6; X is -O-; R⁴, R⁵, R⁶, and R⁷ are each independently selected from the group consisting of hydrogen.

In some embodiments, in the compound represented by formula I or II or the salt thereof, p is selected from the group consisting of 1 and 2, and o is selected from the group consisting of 5 and 6; X is -O-; R⁴, R⁵, R⁶, and R⁷ are each independently selected from the group consisting of hydrogen.

In some embodiments, in the compound represented by formula I or II or the salt thereof, p is selected from the group consisting of 1 and 2, and o is selected from the group consisting of 5 and 6; X is -O-; R⁴, R⁵, R⁶, and R⁷ are each independently selected from the group consisting of hydrogen; L¹ and L² are each independently selected from the group consisting of -C(O)O- and -OC(O)-.

In some other embodiments, in the compound represented by formula I or formula II or the salt thereof, each R³ is independently selected from the group consisting of C₁₋₆ alkyl and C₁₋₆ alkylamino.

In some other embodiments, in the compound represented by formula I or formula II or the salt thereof, each R³ is independently selected from the group consisting of C₁₋₆ alkyl, such as methyl or ethyl.

In certain embodiments, in the compound represented by formula I or formula II or the salt thereof, R³ is selected from the group consisting of C₁₋₆ alkylamino, such as methylamino or dimethylamino.

In certain embodiments, in the compound represented by formula I or formula II or the salt thereof, each R³ is independently selected from the group consisting of halogen, such as fluorine or bromine.

In certain embodiments, in the compound represented by formula I or formula II or the salt thereof, each R³ is independently selected from the group consisting of C₁₋₆ alkoxy, such as methoxy or ethoxy.

In certain embodiments, in the compound represented by formula I or formula II or the salt thereof, each R³ is independently selected from the group consisting of C₃₋₇ cycloalkyl and C₃₋₇ heterocycloalkyl, such as cyclopropyl or epoxyethyl.

In some embodiments, in the compound represented by formula I or II or the salt thereof, L¹ and L² are each independently selected from the group consisting of -C(O)O-, -OC(O)-, and a bond.

Further, the compound represented by formula I or formula II described in the present disclosure is a compound represented by formula III-a or a compound represented by formula III-b: 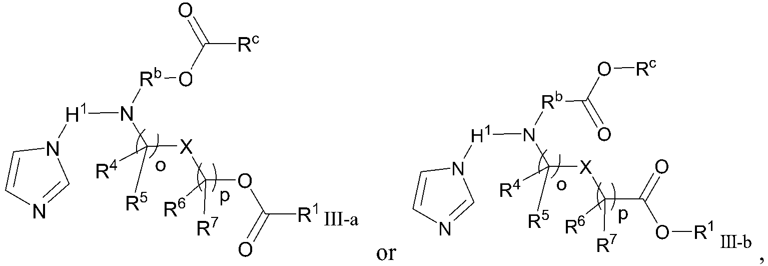 wherein X, H¹, R¹, R⁴-R⁷, R^{b}, R^{c}, o, and p are as defined above.

In some embodiments, in the compound represented by formula I or II or the salt thereof, L¹ and L² are each independently selected from the group consisting of -S(O)_{q}-, -S-S-, -C(O)S-, -SC(O)-, and a bond.

In certain embodiments, in the compound represented by formula I or II or the salt thereof, L¹ and L² are each independently selected from the group consisting of -NR^{a}C(O)-, -C(O)NR^{a}-, -NR^{a}C(O)NR^{a}-, -NR^{a}C(O)O-, and a bond, wherein R^{a} is selected from the group consisting of hydrogen and C₁₋₆ alkyl.

In certain embodiments, in the compound represented by formula I or II or the salt thereof, L¹ and L² are each independently selected from the group consisting of -NR^{a}C(O)NR^{a}-, -NR^{a}C(O)O-, -OC(O)NR^{a}-, and a bond, and R^{a} is selected from the group consisting of hydrogen and C₁₋₆ alkyl.

In another aspect, in some embodiments, in the compound represented by formula I or II, H¹ is selected from the group consisting of C₁₋₆ alkylene, including C₂ alkylene, C₃ alkylene, C₄ alkylene, C₅ alkylene, or C₆ alkylene.

In some embodiments, in the compound represented by formula I or II, H¹ is selected from the group consisting of linear C₁₋₆ alkylene.

In some embodiments, in the compound represented by formula I or II, H¹ is selected from the group consisting of C₃ alkylene, such as linear C₃ alkylene.

In some embodiments, in the compound represented by formula I or II, H¹ is selected from the group consisting of C₄ alkylene, such as linear C₃ alkylene.

In some embodiments, in the compound represented by formula I or II, H¹ is selected from the group consisting of C₅ alkylene, such as linear C₃ alkylene.

In some embodiments, in the compound represented by formula I or II or the salt thereof, p is selected from the group consisting of 3 and 4, and o is selected from the group consisting of 5 and 6; X is -O-; R⁴, R⁵, R⁶, and R⁷ are each independently selected from the group consisting of hydrogen; H¹ is selected from the group consisting of linear C₃ alkylene.

In some embodiments, in the compound represented by formula I or II or the salt thereof, p is selected from the group consisting of 3 and 4, and o is selected from the group consisting of 5 and 6; X is -O-; R⁴, R⁵, R⁶, and R⁷ are each independently selected from the group consisting of hydrogen; H¹ is selected from the group consisting of linear C₃ alkylene; R^{b} is linear C₆₋₈ alkylene.

In the compound represented by formula I or II provided in some embodiments, R^{c} is selected from the group consisting of: 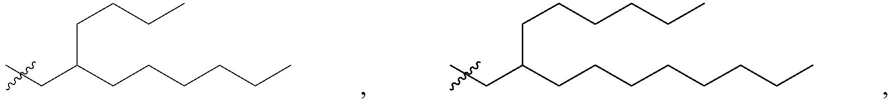 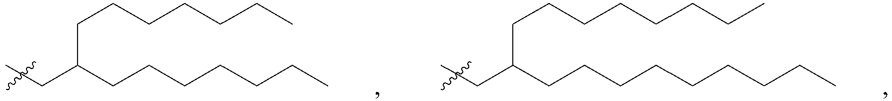 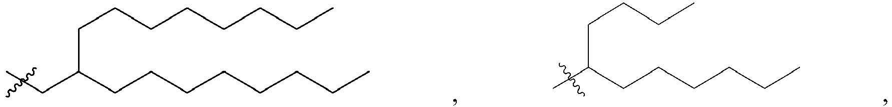 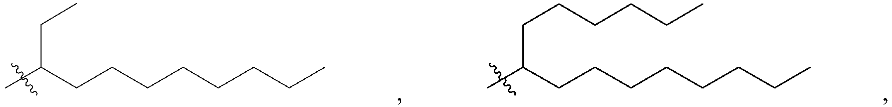 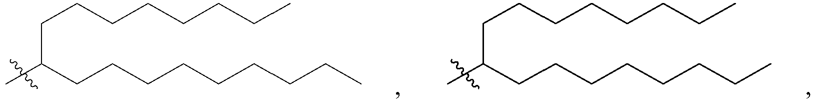 and

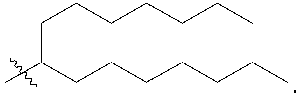

In another aspect, in the compound represented by formula I or II provided in some embodiments, R¹ is selected from the group consisting of: 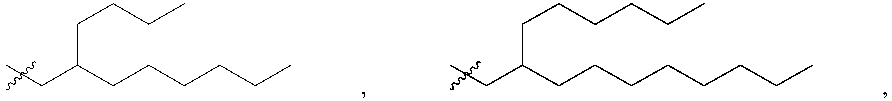 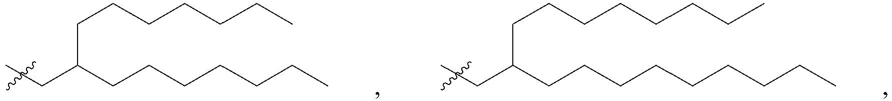 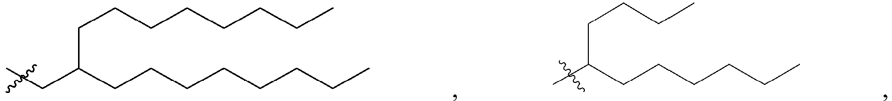 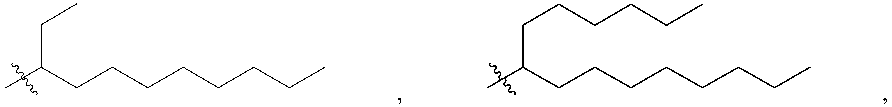 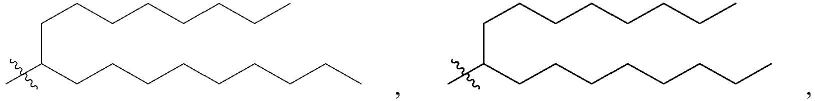 and

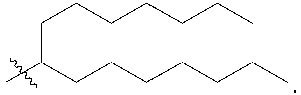

In another aspect, in some embodiments, in the compound represented by formula I or II or the salt thereof, R^{b} is selected from the group consisting of C₁₋₁₂ heteroalkylene, including C₃ heteroalkylene, C₄ heteroalkylene, C₅ heteroalkylene, C₆ heteroalkylene, C₇ heteroalkylene, C₈ heteroalkylene, C₉ heteroalkylene, C₁₀ heteroalkylene, C₁₁ heteroalkylene, or C₁₂ heteroalkylene.

In some embodiments, in the compound represented by formula I or II or the salt thereof, R^{b} is linear C₁₋₁₂ heteroalkylene.

In some embodiments, in the compound represented by formula I or II or the salt thereof, R^{b} is branched C₁₋₁₂ heteroalkylene.

In some embodiments, in the compound represented by formula I or II or the salt thereof, R^{b} is linear C₇ heteroalkylene.

In some embodiments, in the compound represented by formula I or II or the salt thereof, R^{b} is linear C₈ heteroalkylene.

In some embodiments, in the compound represented by formula I or II or the salt thereof, R^{b} is linear C₉ heteroalkylene.

In some embodiments, in the compound represented by formula I or II or the salt thereof, R^{b} is selected from the group consisting of: 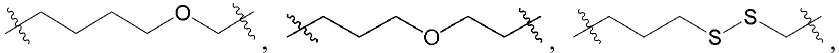 and

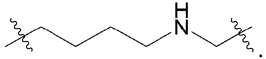

In another aspect, in some embodiments, in the compound represented by formula I or II or the salt thereof, R^{c} is selected from the group consisting of C₁₋₂₄ heteroalkyl, including C₂ heteroalkyl, C₄ heteroalkyl, C₅ heteroalkyl, C₆ heteroalkyl, C₇ heteroalkyl, C₈ heteroalkyl, C₉ heteroalkyl, C₁₀ heteroalkyl, C₁₁ heteroalkyl, C₁₂ heteroalkyl, C₁₃ heteroalkyl, C₁₄ heteroalkyl, C₁₅ heteroalkyl, C₁₆ heteroalkyl, C₁₇ heteroalkyl, C₁₈ heteroalkyl, C₁₉ heteroalkyl, C₂₀ heteroalkyl, C₂₁ heteroalkyl, C₂₂ heteroalkyl, C₂₃ heteroalkyl, or C₂₄ heteroalkyl.

In some embodiments, in the compound represented by formula I or II or the salt thereof, R^{c} is linear C₁₋₁₂ heteroalkyl.

In some embodiments, in the compound represented by formula I or II or the salt thereof, R^{c} is branched C₁₋₁₂ heteroalkyl.

In some embodiments, in the compound represented by formula I or II or the salt thereof, R^{c} is branched C₁₁ heteroalkyl.

In some embodiments, in the compound represented by formula I or II or the salt thereof, R^{c} is branched C₁₇ heteroalkyl.

In some embodiments, in the compound represented by formula I or II or the salt thereof, R^{c} is linear C₉ heteroalkyl.

In some embodiments, in the compound represented by formula I or II or the salt thereof, R^{c} is selected from the group consisting of:

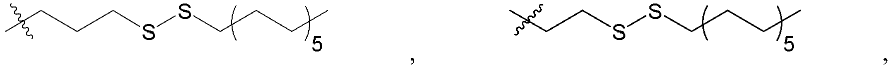

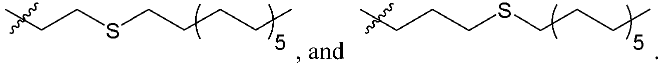

A typical compound of the present disclosure is selected from the group consisting of: 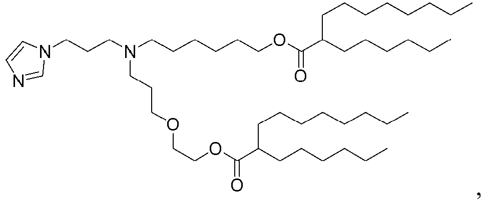 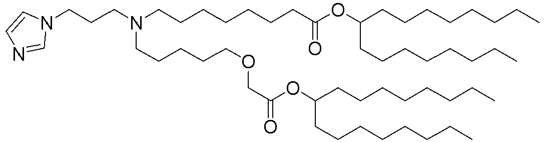 and

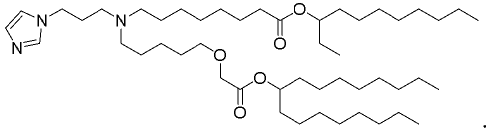

The present disclosure further provides an isotopically substituted form of the aforementioned compound or the salt thereof. In some embodiments, the isotopically substituted form is a deuterium-substituted form.

The present disclosure further provides a lipid particle comprising the aforementioned compound or the salt thereof or the isotopically substituted form thereof. Further, in some embodiments, the lipid particle further comprises an active agent.

In some embodiments, the active agent is selected from the group consisting of a polynucleotide and a nucleic acid (e.g., a ribonucleic acid or a deoxyribonucleic acid). In some embodiments, the active agent is selected from the group consisting of a DNA, an siRNA, and an mRNA.

The present disclosure further provides a pharmaceutical composition comprising the aforementioned lipid particle and a pharmaceutically acceptable excipient. In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The present disclosure further provides use of the aforementioned compound or the salt thereof, or the isotopically substituted form, or the aforementioned lipid particle, or the aforementioned pharmaceutical composition in the manufacture of a medicament for inducing an immune response in a subject.

An *in vitro* activity assay of the aforementioned compound or pharmaceutical composition in the present disclosure shows that the active agent can be specifically delivered to immune cells such as T cells, and moreover, the delivered active agent exhibits a better protein expression ability in immune cells such as T cells.

In addition, specific delivery was also observed in other cell types, such as NK cells, macrophages, dendritic cells, and monocytes.

The present disclosure further provides use of the aforementioned compound or the salt thereof, or the isotopically substituted form, or the aforementioned lipid particle, or the aforementioned pharmaceutical composition in the manufacture of a medicament for preventing and/or treating a disease or disorder associated with polypeptide overexpression.

The present disclosure further provides use of the aforementioned compound or the salt thereof, or the isotopically substituted form, or the aforementioned lipid particle, or the aforementioned pharmaceutical composition in the manufacture of a medicament for preventing and/or treating a disease or disorder associated with insufficient polypeptide expression.

In some embodiments, the disease or disorder includes, but is not limited to, cancer, infectious diseases, autoimmune diseases, neurodegenerative diseases, and inflammation.

The present disclosure further provides a method for preventing and/or treating a disease or disorder associated with induction of an immune response in a subject, comprising administering to a subject in need thereof the aforementioned compound or the salt thereof or the isotopically substituted form thereof, or the aforementioned lipid particle, or the aforementioned pharmaceutical composition.

The present disclosure further provides a method for preventing and/or treating a disease or disorder associated with polypeptide overexpression, comprising administering to a subject in need thereof the aforementioned compound or the salt thereof or the isotopically substituted form thereof, or the aforementioned lipid particle, or the aforementioned pharmaceutical composition.

The present disclosure further provides a method for preventing and/or treating a disease or disorder associated with insufficient polypeptide expression, comprising administering to a subject in need thereof the aforementioned compound or the salt thereof or the isotopically substituted form thereof, or the aforementioned lipid particle, or the aforementioned pharmaceutical composition.

In another aspect, the present disclosure further provides the aforementioned compound or the salt thereof or the isotopically substituted form thereof, or the aforementioned lipid particle, or the aforementioned pharmaceutical composition for use in the prevention and/or treatment of a disease or disorder associated with induction of an immune response in a subject.

The present disclosure further provides the aforementioned compound or the salt thereof or the isotopically substituted form thereof, or the aforementioned lipid particle, or the aforementioned pharmaceutical composition for use in the prevention and/or treatment of a disease or disorder associated with polypeptide overexpression.

The present disclosure further provides the aforementioned compound or the salt thereof or the isotopically substituted form thereof, or the aforementioned lipid particle, or the aforementioned pharmaceutical composition for use in the prevention and/or treatment of a disease or disorder associated with insufficient polypeptide expression.

The present disclosure further provides use of the aforementioned compound or the salt thereof, or the isotopically substituted form, or the aforementioned lipid particle, or the aforementioned pharmaceutical composition in the manufacture of a medicament for preventing and/or treating cancer, infectious diseases, autoimmune diseases, neurodegenerative diseases, or inflammation.

The present disclosure further provides a method for preventing and/or treating cancer, infectious diseases, autoimmune diseases, neurodegenerative diseases, or inflammation, comprising administering to a subject in need thereof the aforementioned compound or the salt thereof or the isotopically substituted form thereof, or the aforementioned lipid particle, or the aforementioned pharmaceutical composition.

The present disclosure further provides the aforementioned compound or the salt thereof or the isotopically substituted form thereof, or the aforementioned lipid particle, or the aforementioned pharmaceutical composition for use in the prevention and/or treatment of cancer, infectious diseases, autoimmune diseases, neurodegenerative diseases, or inflammation.

The salt of the compound described in the present disclosure includes "acid" addition salts and "base" addition salts. For example, salts formed by acid-base reactions with basic groups (amino) are included, and the acid includes organic acids or inorganic acids. In addition, the salt of the compound also includes salts formed by quaternization with basic groups (amino); the quaternizing reagent includes linear or branched chlorinated hydrocarbons.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures, and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof fall within the scope of the present disclosure. The compounds of the present disclosure containing asymmetric carbon atoms may be isolated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., formation of a carbamate from an amine).

In the chemical structures of the compounds of the present disclosure, the bond " 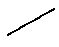 " indicates an unspecified configuration; that is, if chiral isomers exist in the chemical structures, the bond " 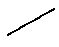 " may be " 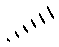 " or " 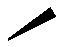 ", or includes both the configurations " 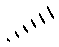 " and " 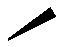 " simultaneously. In the chemical structures of the compounds of the present disclosure, the bond " 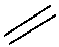 " does not specify a configuration; that is, it may be a Z configuration or an E configuration, or includes both configurations simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine, and lactam-lactim isomerization. For example, between the following A and B:

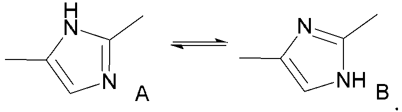

All tautomeric forms fall within the scope of the present disclosure. The names of the compounds do not exclude any tautomers.

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds in examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compounds. Each available hydrogen atom linked to a carbon atom may be independently replaced with a deuterium atom. Those skilled in the art can synthesize the deuterated forms of the compounds by reference to the relevant literature. Commercially available deuterated starting materials can be used in preparing the deuterated compounds, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

### Terms and Definitions

"Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity.

"Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

"Effective amount" or "therapeutically effective amount", as described in the present disclosure, includes an amount sufficient to ameliorate or prevent a symptom of a medical disease or a disorder. An effective amount also means an amount sufficient to allow or facilitate diagnosis. The effective amount for a particular patient or veterinary subject may vary depending on factors such as the disorder to be treated, the general health of the patient, the method, route, and dose of administration, and the severity of side effects. An effective amount may be the maximum dose or administration regimen to avoid significant side effects or toxic effects.

The term "nucleic acid" is a polymer consisting of nucleotides, such as deoxyribonucleotides (DNA) or ribonucleotides (RNA).

The term "oligonucleotide" describes a single-stranded or double-stranded nucleotide polymer that is 2 to 100 nucleotides in length. "Polynucleotide" refers to a single-stranded or double-stranded polymer consisting of nucleotide monomers. In some embodiments, the polynucleotide consists of 100 or more nucleotides.

In some other embodiments, exemplary polynucleotides include, but are not limited to, deoxyribonucleotides (DNAs) and ribonucleic acids (RNAs), including messenger RNAs, RNAi-inducing agents, shRNAs, siRNAs, miRNAs, antisense RNAs, and analogs.

The terms "polypeptide", "peptide", and "protein" are used interchangeably herein to refer to a polymer of amino acid residues. The terms apply to amino acid polymers in which one or more amino acid residues are artificial chemical analogs of corresponding naturally occurring amino acids, as well as to naturally occurring amino acid polymers.

"Alkyl" refers to saturated aliphatic hydrocarbon groups, including linear and branched alkyl groups. In some embodiments, alkyl groups comprise 1-4 carbon atoms and are also known as C₁₋₄ alkyl. In some embodiments, alkyl groups comprise 10-22 carbon atoms and are also known as C₁₀₋₂₂ alkyl. In some embodiments, alkyl groups comprise 4-22 carbon atoms and are also known as C₄₋₂₂ alkyl.

An alkyl group may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkyl is substituted with one or more groups selected from the group consisting of halogen, hydroxy, oxo, cyano, amino, C₁₋₆ alkyl, C₁₋₆ alkoxy, and 3- to 7-membered cycloalkyl.

"Alkoxy" refers to -O-(alkyl), wherein the alkyl is as defined above. Non-limiting examples of alkoxy groups include methoxy or ethoxy. Alkoxy may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkoxy is substituted with one or more groups selected from the group consisting of halogen, hydroxy, oxo, cyano, amino, C₁₋₆ alkyl, C₁₋₆ alkoxy, and 3- to 7-membered cycloalkyl.

"Alkylamino" refers to -NH(alkyl) or -N(alkyl)₂, wherein the alkyl is as defined above. Exemplary alkylamino groups include C₁₋₆ alkylNH- or di(C₁₋₆ alkyl)N-, such as methylamino or dimethylamino. Alkylamino may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkylamino is substituted with one or more groups selected from the group consisting of halogen, hydroxy, oxo, cyano, amino, C₁₋₆ alkyl, C₁₋₆ alkoxy, and 3- to 7-membered cycloalkyl.

"Heteroalkyl" refers to a linear or branched alkyl group that preferably comprises 1 to 14 carbon atoms and more preferably comprises 2 to 10 carbon atoms in the chain, wherein one or more carbon atoms are substituted with heteroatoms selected from the group consisting of S, O, and N, including the moiety -O-O- or -S-S-. Exemplary heteroalkyl groups include 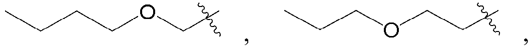 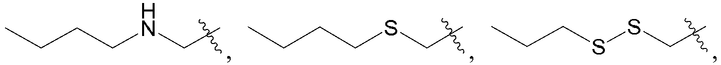 or the like.

Heteroalkyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the heteroalkyl is substituted with one or more groups selected from the group consisting of halogen, hydroxy, oxo, cyano, amino, C₁₋₆ alkyl, C₁₋₆ alkoxy, and 3- to 7-membered cycloalkyl.

"Alkenyl" refers to unsaturated aliphatic hydrocarbon groups, including linear and branched alkenyl groups. In some embodiments, alkenyl groups comprise 2-4 carbon atoms and are also known as C₂₋₄ alkenyl. In some embodiments, alkenyl groups comprise 10-22 carbon atoms and are also known as C₁₀₋₂₂ alkenyl. In some embodiments, alkenyl groups comprise 4-22 carbon atoms and are also known as C₄₋₂₂ alkenyl. Exemplary alkenyl groups include ethenyl, propenyl, n-butenyl, isobutenyl, 3-methylbut-2-enyl, n-pentenyl, heptenyl, octenyl, cyclohexyl-butenyl, and decenyl.

Alkenyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkenyl is substituted with one or more groups selected from the group consisting of halogen, hydroxy, oxo, cyano, amino, C₁₋₆ alkyl, C₁₋₆ alkoxy, and 3- to 7-membered cycloalkyl.

For the definition of "heteroalkenyl", see the definition of "heteroalkyl". Heteroalkenyl refers to a linear or branched alkenyl group in which one or more carbon atoms are substituted with heteroatoms selected from the group consisting of S, O, and N, including the moiety -O-O- or -S-S-.

"Monovalent group" refers to a group formed by "formally" eliminating a monovalent atom or group from a compound. "-ylene" refers to a group formed by "formally" eliminating two monovalent atoms or groups of atoms or one divalent atom or group of atoms from a compound.

The term "alkylene" refers to the remainder of an alkane molecule after 2 hydrogen atoms are removed from the alkane molecule. In some embodiments, alkylene groups comprise 1-4 carbon atoms and are also known as C₁₋₄ alkylene. In some embodiments, alkyl groups comprise 10-22 carbon atoms and are also known as C₁₀₋₂₂ alkylene. In some embodiments, alkyl groups comprise 4-22 carbon atoms and are also known as C₄₋₂₂ alkylene.

Alkylene may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkylene is substituted with one or more groups selected from the group consisting of halogen, hydroxy, oxo, cyano, amino, C₁₋₆ alkyl, C₁₋₆ alkoxy, and 3- to 7-membered cycloalkyl.

The term "heteroalkylene" refers to the remainder of a heteroalkane molecule after 2 hydrogen atoms are removed from the heteroalkane molecule. In some embodiments, heteroalkylene comprises 1-14 carbon atoms and is also known as C₁₋₁₄ heteroalkylene. In some embodiments, heteroalkylene comprises 2-10 carbon atoms and is also known as C₂₋₁₀ heteroalkylene. In some other embodiments, one or more carbon atoms in the chain of heteroalkyl are substituted with a heteroatom selected from the group consisting of S, O, and N, including the moiety -O-O- or -S-S-. Exemplary heteroalkylene groups include 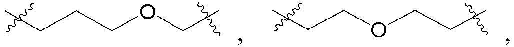 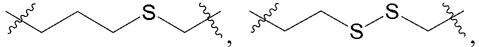 or the like.

Heteroalkylene may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl.

Likewise, the definitions of "alkyleneoxy" and "alkenylene" are the same as the definition of "alkylene".

The term "cycloalkyl" refers to a saturated or partially unsaturated, monocyclic or polycyclic hydrocarbon substituent, and the cycloalkyl ring contains 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the cycloalkyl is substituted with one or more groups selected from the group consisting of halogen, hydroxy, oxo, cyano, amino, C₁₋₆ alkyl, C₁₋₆ alkoxy, and 3- to 7-membered cycloalkyl.

The term "heterocycloalkyl" refers to a saturated or partially unsaturated, monocyclic or polycyclic hydrocarbon substituent containing 3 to 7 ring atoms, wherein one or more ring atoms are heteroatoms selected from the group consisting of nitrogen, oxygen, and S(O)ₘ (where m is an integer from 0 to 2), but a ring moiety of -O-O-, -O-S-, or -S-S- is excluded, and the other ring atoms are carbon atoms. It preferably contains 3 to 7 ring atoms, of which 1 to 4 are heteroatoms. Non-limiting examples of "heterocycloalkyl" groups include 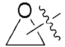 or 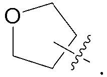 Heterocycloalkyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the heterocycloalkyl is substituted with one or more groups selected from the group consisting of halogen, hydroxy, oxo, cyano, amino, C₁₋₆ alkyl, C₁₋₆ alkoxy, and 3- to 7-membered cycloalkyl.

The terms "branched alkyl" and "branched alkenyl" refer to alkyl and alkenyl groups in which one carbon atom is linked to at least 2 other carbon atoms, and a cyclic group, such as a spiro ring, is not formed. Examples of C₆ alkyl groups include 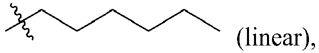 or 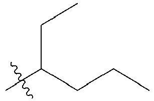 and

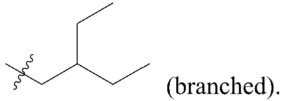

The term "hydroxy" refers to the -OH group.

The term "halogen" refers to fluorine, chlorine, bromine, or iodine.

The term "amino" refers to -NH₂.

The term "cyano" refers to -CN.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

### BRIEF DESCRIPTION OF THE DRAWINGS

FIG. 1: the measured fluorescence intensities for the non-immune cell *in vitro* transfection efficiency of the lipid nanoparticle compositions of the present disclosure (ns denotes P > 0.05, ** denotes P < 0.01, and *** denotes P < 0.001).
FIG. 2: the relative fluorescence intensities for the non-immune cell *in vitro* transfection efficiency of the lipid nanoparticle compositions of the present disclosure (ns denotes P > 0.05, ** denotes P < 0.01, and *** denotes P < 0.001).

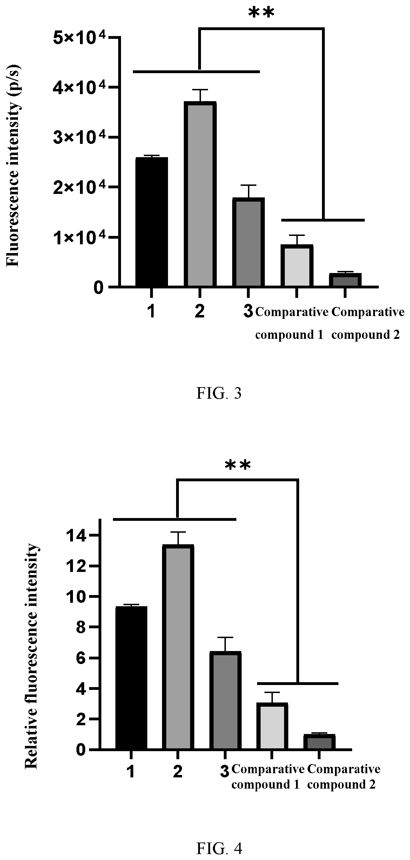

FIG. 3: the measured fluorescence intensities for the immune cell *in vitro* transfection efficiency of the lipid nanoparticle compositions of the present disclosure (** denotes P < 0.01).
FIG. 4: the relative fluorescence intensities for the immune cell *in vitro* transfection efficiency of the lipid nanoparticle compositions of the present disclosure (** denotes P < 0.01).
FIG. 5: the proportion of positive cells in T cells, as determined by flow cytometry (** denotes p < 0.01, and *** denotes p < 0.001).
FIG. 6: the proportion of positive cells in NK cells, as determined by flow cytometry (** denotes p < 0.01, and *** denotes p < 0.001).
FIG. 7: the proportion of positive cells in macrophages, as determined by flow cytometry (** denotes p < 0.01, and *** denotes p < 0.001).
FIG. 8: the proportion of positive cells in dendritic cells, as determined by flow cytometry (** denotes p < 0.01, and *** denotes p < 0.001).
FIG. 9: the proportion of positive cells in monocytes, as determined by flow cytometry (** denotes p < 0.01, and *** denotes p < 0.001).
FIG. 10: the fluorescence intensities of mouse spleen tissues, as measured by IVIS (ns denotes P > 0.05, * denotes P < 0.05, and ** denotes P < 0.01).
FIG. 11: the fluorescence intensities of mouse liver tissues, as measured by IVIS (ns denotes P > 0.05, * denotes P < 0.05, and ** denotes P < 0.01).

### DETAILED DESCRIPTION

The present disclosure is further described below with reference to examples, but these examples are not intended to limit the scope of the present disclosure.

Experimental methods without specific conditions indicated in the examples of the present disclosure were generally conducted under conventional conditions, or conditions recommended by the manufacturers of the starting materials or commercial products. Reagents without specific sources indicated were commercially available conventional reagents.

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument, with dimethyl sulfoxide-D6 (DMSO-*d₆*), chloroform-D (CDCl₃), or methanol-D4 (methanol-*d₄*) as the solvent and tetramethylsilane (TMS) as the internal standard.

The HPLC analyses were performed using an Agilent 1100 high pressure liquid chromatograph, a GAS15B DAD ultraviolet detector, and a Water Vbridge C18 150 × 4.6 mm 5 µm chromatography column.

The MS analyses were performed using an Agilent 6120 triple quadrupole mass spectrometer, a G1315D DAD detector, and a Waters Xbridge C18 4.6 × 50 mm, 5 µm chromatography column, with scanning performed in positive/negative ion mode with a mass scan range of 80-1200.

The thin-layer chromatography silica gel plates used were Yantai Huanghai HSGF254 silica gel plates. The silica gel plates used in the thin-layer chromatography (TLC) analyses had a layer thickness of 0.2 mm ± 0.03 mm, and those used in the thin-layer chromatography separation and purification had a layer thickness of 0.4 mm-0.5 mm.

The flash column purification system used was Combiflash Rf150 (TELEDYNE ISCO) or Isolara one (Biotage).

In the normal-phase column chromatography, a 200-300 mesh or 300-400 mesh Yantai Huanghai silica gel was generally used as the carrier, or a Changzhou Santai pre-fill ultrapure normal-phase silica gel column (40-63 µm, 60 g, 24 g, 40 g, 120 g, or other specifications) was used.

The known starting materials in the present disclosure may be synthesized by using or following methods known in the art, or may be purchased from Shanghai Titan Scientific, ABCR GmbH & Co. KG, Acros Organics, Aldrich Chemical Company, Accela ChemBio Inc., and Bide Pharmatech, among others.

In the examples, the reactions can all be performed in a nitrogen atmosphere unless otherwise specified.

The nitrogen atmosphere means that the reaction flask is connected to a balloon containing about 1 L of nitrogen.

The hydrogen atmosphere means that the reaction flask is connected to a balloon containing about 1 L of hydrogen.

The hydrogen gas was prepared by a QPH-1L hydrogen generator from Shanghai Quan Pu Scientific Instruments Inc.

The nitrogen atmosphere or the hydrogenation atmosphere was generally formed by 3 cycles of vacuumization and nitrogen or hydrogen filling.

In the examples, the solutions were aqueous solutions unless otherwise specified.

In the examples, the reaction temperature was room temperature, i.e., 20 °C-30 °C, unless otherwise specified.

The monitoring of the reaction progress in the examples was performed using thin-layer chromatography (TLC). The developing solvents used in the reactions, the eluent systems used in the column chromatography purification of the compounds, the developing solvent systems used in the thin-layer chromatography, and the volume ratio of the solvents were adjusted depending on the polarity of the compounds, or a small amount of basic or acidic reagents such as triethylamine and acetic acid could be added for adjustment.

### Example 1: Preparation of Compound 1

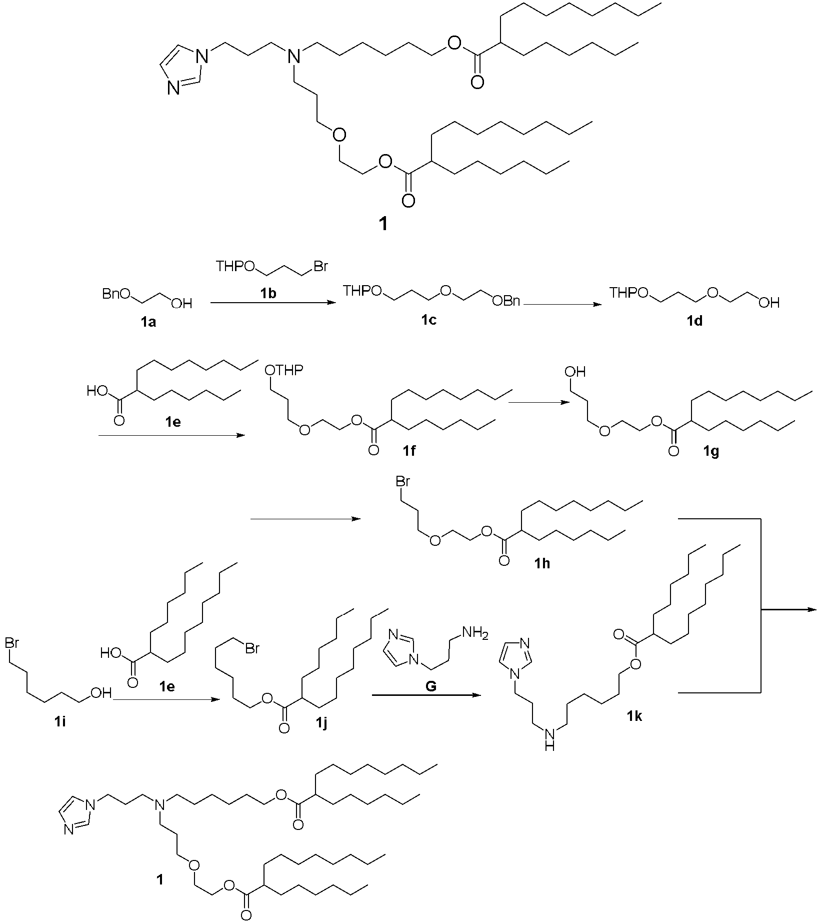

### Step 1: Preparation of compound 1c

Compound 1a (5 g, 32.85 mmol, purchased from Adamas) and compound 1b (14.66 g, 65.71 mmol, purchased from Leyan) were dispersed in a 50% aqueous NaOH solution (8 M, 69.81 mL), and the dispersion was stirred. The mixture was cooled to 0 °C. After tetrabutylammonium hydrogen sulfate (1.12 g, 3.29 mmol) was added, the mixture was heated to 60 °C and left to react for 16 h. After the reaction mixture was cooled to 0 °C, water (100 mL) was added, and ethyl acetate (100 mL) was then added. The aqueous phase was extracted with ethyl acetate (50 mL × 2). The organic phases were combined, washed with saturated brine (50 mL), dried over anhydrous sodium sulfate, filtered, and concentrated to dryness, and the residue was purified by column chromatography (PE:EA = 0-20%) to give compound 1c (7.69 g, yield: 79.51%).

### Step 2: Preparation of compound 1d

Compound 1c (7.69 g, 26.12 mmol) was dissolved in MeOH (80 mL), and the solution was stirred. Palladium on carbon (10 wt.%, 50% water, 1.83 g, 13.06 mmol) was added. The system was purged three times with hydrogen and then pressurized to 60 psi, heated to 45 °C, and left to react for 16 h. The reaction mixture was filtered through diatomaceous earth. The filter cake was washed with methanol, and the filtrate was concentrated to dryness to give crude compound 1d (5.54 g), which was directly used in the next step.

### Step 3: Preparation of compound 1f

Compound 1d (5.34 g, 26.14 mmol), compound 1e (8.04 g, 31.37 mmol, purchased from Bide Pharmatech), and DMAP (3.19 g, 26.14 mmol) were dissolved in DCM (60 mL), and the solution was stirred at 0 °C. After EDCI (7.52 g, 39.21 mmol) was added, the mixture was naturally warmed to room temperature and left to react for 16 h. DCM (50 mL) and water (50 mL) were added. The organic phase was washed with saturated sodium bicarbonate (25 mL × 2). The organic phases were combined, dried over anhydrous sodium sulfate, filtered, and concentrated to dryness, and the residue was purified by column chromatography (PE:EA = 0-10%) to give compound 1f (10.01 g, yield: 86.50%).

¹H NMR (400 MHz, CDCl3): δ 4.59-4.54 (m, 1H), 4.24-4.17 (m, 2H), 3.89-3.76 (m, 2H), 3.63-3.59 (m, 2H), 3.58-3.52 (m, 2H), 3.51-3.43 (m, 2H), 2.39-2.29(m, 1H), 1.91-1.75 (m, 3H), 1.74-1.66 (m, 1H), 1.65-1.47 (m, 6H), 1.46-1.36 (m, 2H), 1.33-1.15 (m, 20H), 0.92-0.80 (m, 6H).

### Step 4: Preparation of compound 1g

Compound 1f (1 g, 2.26 mmol) was dissolved in hydrochloric acid/dioxane (4 M, 10.05 mL), and the solution was stirred at room temperature for 2 h. The reaction mixture was concentrated to dryness, and the residue was purified by column chromatography (PE:EA = 0-20%) to give compound 1g (203 mg, yield: 25.06%).

¹H NMR (400 MHz, CDCl₃): δ 4.27-4.19 (m, 2H), 3.76 (t, J=5.6 Hz, 2H), 3.68-3.61 (m, 4H), 2.40-2.30 (m, 1H), 2.29-2.20 (brs, 1H), 1.87-1.78 (m, 2H), 1.66-1.51(m, 3H), 1.50-1.35 (m, 2H), 1.35-1.15 (m, 19H), 0.92-0.80 (m, 6H).

### Step 5: Preparation of compound 1h

Compound 1g (0.16 g, 446.24 µmol) and triphenylphosphine (175.56 mg, 669.36 µmol) were dissolved in dichloromethane (2 mL), and the solution was stirred at 0 °C. After carbon tetrabromide (221.98 mg, 669.36 µmol) was added in batches, the mixture was naturally warmed to room temperature and left to react for 2 h. The reaction mixture was concentrated to dryness, and the residue was purified by column chromatography (PE:EA = 0-5%) to give compound 1h (149 mg, yield: 79.23%).

¹H NMR (400 MHz, CDCl₃): δ 4.25-4.19 (m, 2H), 3.66-3.61 (m, 2H), 3.58 (t, J=6 Hz, 2H), 3.49 (t, J=6.4 Hz, 2H), 2.40-2.29 (m, 1H), 2.13-2.04 (m, 2H), 1.65-1.52(m, 2H), 1.50-1.36 (m, 2H), 1.34-1.15 (m, 20H), 0.92-0.79 (m, 6H).

### Step 6: Preparation of compound 1j

Compound 1i (5 g, 27.61 mmol, purchased from Accela ChemBio), compound 1e (8.50 g, 33.14 mmol), and DMAP (3.37 g, 27.61 mmol) were dissolved in DCM (50 mL), and the solution was stirred at 0 °C. After EDCI (7.94 g, 41.42 mmol) was added, the mixture was naturally warmed to room temperature and left to react for 16 h. 2 M HCl (10 mL) was added, and the aqueous phase was extracted with dichloromethane (20 mL × 2). The organic phases were combined, dried over anhydrous sodium sulfate, filtered, and concentrated to dryness, and the residue was purified by column chromatography (PE:EA= 0-10%) to give compound 1j (5.96 g, yield: 51.45%).

¹ H NMR (400 MHz, CDCl3): δ 4.06 (t, J=6.4 Hz, 2H), 3.40 (t, J=6.4 Hz, 2H), 2.35-2.25 (m, 1H), 1.91-1.81 (m, 2H), 1.68-1.52 (m, 5H), 1.51-1.34 (m, 5H), 1.33-1.18(m, 20H), 0.92-0.79 (m, 6H).

### Step 7: Preparation of compound 1k

Compound 1j (2 g, 4.77 mmol) and compound G (2.98 g, 23.84 mmol, purchased from Adamas) were dissolved in ethanol (20 mL), and the solution was stirred at 78 °C for 16 h. The reaction mixture was concentrated to dryness, and the residue was purified by column chromatography (DCM:MeOH = 0-15%) to give compound 1k (1.13 g, yield: 51.11%).

¹H NMR (400 MHz, CDCl₃): δ 7.46 (s, 1H), 7.04 (s, 1H), 6.91 (s, 1H), 4.11-3.98 (m, 4H), 2.64-2.52 (m, 4H), 2.35-2.24 (m, 1H), 1.96-1.86 (m, 2H), 1.68-1.51 (m, 4H), 1.50-1.32 (m, 9H), 1.31-1.17 (m, 20H), 0.92-0.81 (m, 6H).

### Step 8: Preparation of compound 1

Compound 1k (0.5 g, 1.08 mmol) and compound 1h (413.10 mg, 980.18 µmol) were dissolved in acetonitrile (4.5 mL) and cyclopentyl methyl ether (4.5 mL). After potassium iodide (178.98 mg, 1.08 mmol) and potassium carbonate (541.88 mg, 3.92 mmol) were added, the mixture was heated to 86 °C and left to react for 16 h. The reaction mixture was filtered. After the filter cake was washed with acetonitrile, the filtrate was concentrated to dryness, and the residue was purified by reversed-phase C4 column chromatography (H₂O/ACN = 0-100%) to give compound 1 (156.9 mg, yield: 19.90%).

¹H NMR (400 MHz, CDCl₃): δ 7.45 (s, 1H), 7.04 (s, 1H), 6.91 (s, 1H), 4.24-4.18 (m, 2H), 4.05 (t, J=6.8 Hz, 2H), 3.97 (t, J=7.2 Hz, 2H), 3.62-3.57 (m, 2H), 3.46 (t, J=6.4Hz, 2H), 2.49-2.41 (m, 2H), 2.40-2.25 (m, 6H), 1.92-1.83 (m, 2H), 1.75-1.50 (m, 8H), 1.49-1.33 (m, 8H), 1.32-1.17 (m, 42H), 0.91-0.81 (m, 12H).

### Example 2: Preparation of Compound 2

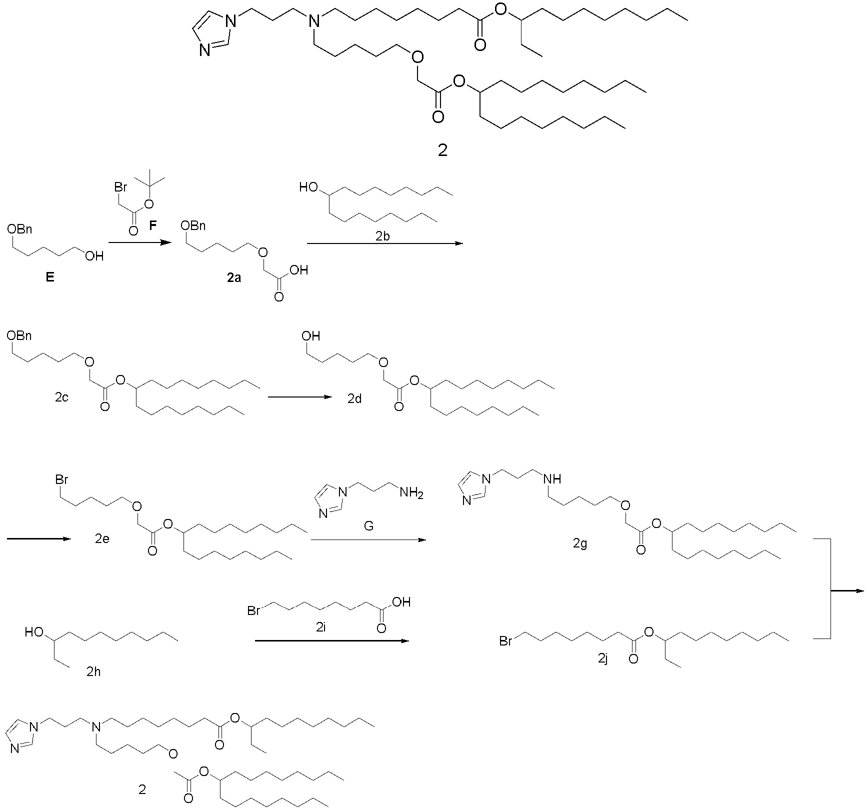

### Step 1: Preparation of compound 2a

Water (46.8 mL) was added to a 500 mL three-necked flask. After the temperature was lowered to below 5 °C using an ice-water bath, sodium hydroxide (46.8 g, 1170 mmol) was slowly added, during which the internal temperature was kept below 15 °C. After sodium hydroxide was completely dissolved, toluene (46.8 mL) and compound E (9.71 g, 50.0 mmol, purchased from Adamas) were added, and the mixture was stirred at room temperature for 1 h. The mixture was then cooled to below 5 °C in an ice-water bath, and compound F (34.1 g, 175 mmol, purchased from Adamas) was slowly added dropwise. The mixture was heated in an oil bath until the external temperature was 25 °C, and the mixture was stirred for 24 h. The reaction was cooled to below 5 °C. Water (200 mL) was added, and extraction was performed with methyl tert-butyl ether (100 mL × 3). The aqueous phase was cooled to below 5 °C in an ice-water bath, and concentrated hydrochloric acid was slowly added until the pH value was about 3.

Extraction was then performed with DCM (100 mL × 5). The DCM phases were combined, washed with saturated brine, dried over anhydrous sodium sulfate, and concentrated under reduced pressure, and the residue was purified by column chromatography (EA/PE = 0-100%) to give compound 2a (11.97 g, yield: 94%).

¹H NMR (400 MHz, CDCl₃): *δ* 8.50 (brs, 1H), 7.36-7.26 (m, 5H), 4.51 (s, 2H), 4.13-4.10 (m,2H), 3.56 (t, 2H), 3.48 (t, 2H), 1.67-1.61 (m, 4H), 1.50-1.43 (m, 2H).

### Step 2: Preparation of compound 2c

Compound 2a (9.3 g, 36.86 mmol), compound 2b (8.51 g, 33.17 mmol, purchased from Admas), and DCM (186 mL) were added to a 100 mL single-necked flask, and the system was cooled to 0 °C in a nitrogen atmosphere. DMAP (4.50 g, 36.86 mmol) was added, and the mixture was stirred for 10 min. EDCI (8.48 g, 44.23 mmol) was slowly added to the reaction mixture. After the addition, the mixture was naturally warmed to room temperature and left to react for 16 h. The reaction mixture was cooled to 0 °C, and water (186 mL) was added. After 5 min of stirring, the aqueous phase was extracted with DCM (100 mL × 2). The organic phases were combined, washed with a saturated NaHCO₃ solution (200 mL) and with a NaCl solution (200 mL), dried, and concentrated under reduced pressure, and the residue was separated and purified by column chromatography (PE:EA = 100:0 to 95:5) to give compound 2c (13.5 g, yield: 74.63%).

¹H NMR (400 MHz, CDCl3): δ (ppm) 7.34-7.27 (m, 4H), 4.99-4.94 (m, 1H), 4.49 (s, 2H), 4.03 (s, 2H), 3.54-3.46 (m, 4H), 1.68-1.61 (m, 4H), 1.53-1.42 (m, 8H), 1.25-1.24 (m, 21H), 0.89-0.86 (m, 8H).

### Step 3: Preparation of compound 2d

Compound 2c (13.5 g, 27.51 mmol) was dissolved in THF (108 mL), and Pd(OH)₂/C (3.7 g, 50% Wt) was added. The system was purged 3 times with hydrogen, heated to 30 °C, and left to react for 48 h. Analysis showed that the starting material had been completely reacted. The reaction mixture was filtered under vacuum, and the filtrate was concentrated to dryness. The residue was separated and purified by column chromatography (PE:EA = 100:0 to 60:10) to give compound 2d (8.07 g, yield: 74.63%).

MS m/z (ESI): 423.3 [M+23]⁺.

### Step 4: Preparation of compound 2e

Compound 2d (3 g, 7.49 mmol) and DCM (24 mL) were added to a 100 mL single-necked flask, and the system was cooled to 0 °C in a nitrogen atmosphere. Carbon tetrabromide (4.37 g, 13.18 mmol) was added, and the mixture was stirred for 10 min. Triphenylphosphine (3.14 g, 11.98 mmol) was added in batches to the reaction mixture. After the addition, the mixture was naturally warmed to room temperature and left to react for 16 h. Analysis showed that the reaction had been complete. Silica gel (18 g, 100-200 mesh) was added, and the mixture was concentrated to dryness by rotary evaporation to give a powder. The powder was separated and purified by column chromatography (PE:EA = 100:0 to 90:10) to give compound 2e (3.2 g, yield: 92.19%).

¹H NMR (400 MHz, CDCl3): δ (ppm) 4.99-4.93 (m, 1H), 4.04 (s, 2H), 3.53 (t, J = 6 Hz, 2H), 3.41 (t, J = 6.8 Hz, 2H), 1.93-1.86 (m, 2H), 1.64-1.53 (m, 8H), 1.29-1.25 (m, 24H), 0.87(t, J = 6.4 Hz, 6H).

### Step 5: Preparation of compound 2g

Compound 2e (3.1 g, 6.69 mmol), compound G (2.51 g, 20.06 mmol), and ethanol (31 mL) were added to a 100 mL single-necked flask, and the system was purged 3 times with nitrogen, heated to 65 °C, and left to react for 16 h. The reaction mixture was concentrated to dryness, and the residue was separated and purified by column chromatography (DCM:MeOH = 100:0 to 90:10) to give compound 2g (1.2 g, yield: 35.34%).

MS m/z (ESI): 508.4 [M+1]⁺.

### Step 6: Preparation of compound 2j

Compound 2h (1.0 g, 5.80 mmol, purchased from Bide Pharmatech), compound 2i (1.94 g, 8.71 mmol, purchased from Accela ChemBio), and DCM (20 mL) were added to a 100 mL single-necked flask, and the system was cooled to 0 °C. DMAP (70.90 mg, 580.36 µmol), EDCI (1.67 g, 8.71 mmol), and DIEA (2.25 g, 17.41 mmol) were sequentially added. After the addition, the mixture was naturally warmed to room temperature and left to react for 16 h. The reaction mixture was cooled to 0 °C, and H₂O (20 mL) was added. After stirring, the organic phase was washed with water, dried, and concentrated under reduced pressure, and the residue was separated and purified by column chromatography (PE:EA = 100:0 to 90:10) to give compound 2j (1.4 g, yield: 63.92%).

¹H NMR (400 MHz, CDCl₃): δ (ppm) 4.84-4.78 (m, 1H), 3.4 (t, J = 7.2 Hz, 2H), 2.34 (t, J = 7.6 Hz, 2H), 1.87-1.83 (m, 2H), 1.63-1.43 (m, 10H), 1.35-1.26 (m, 14H), 0.89-0.85(m, 6H).

### Step 7: Preparation of compound 2

Compound 2g (592.02 mg, 1.17 mmol), compound 2j (400.00 mg, 1.06 mmol), potassium iodide (193.54 mg, 1.17 mmol, 62.03 µL), and potassium carbonate (585.95 mg, 4.24 mmol, 255.87 µL) were added to a 25 mL single-necked flask, and acetonitrile (6 mL) and cyclopentyl methyl ether (6 mL) were added in a nitrogen atmosphere. The system was purged 3 times with nitrogen, heated to 86 °C, and left to react. Analysis showed that the reaction had been complete. The reaction mixture was cooled to room temperature, filtered under vacuum to remove insoluble substances, and concentrated to dryness, and the residue was purified by reversed-phase C4 column chromatography (water:acetonitrile = 100:0 to 0:100) to give compound 2 (115 mg, yield: 13.49%).

¹H NMR (400 MHz, CDCl₃): δ (ppm) 7.46 (s, 1H), 7.04 (s, 1H), 6.91 (s, 1H), 4.99-4.93 (m, 1H), 4.84-4.78 (m, 1H), 4.04 (s, 2H), 3.98(t, J = 6.8 Hz, 2H), 3.53(t, J = 6.2 Hz, 2H), 2.37-2.34 (m, 6H), 2.29(t, J =7.6 Hz, 2H), 1.91-1.84 (m, 2H), 1.67-1.52 (m, 14H), 1.38-1.25 (m, 46H), 0.89-0.86(m, 12H).

### Example 3: Preparation of Compound 3

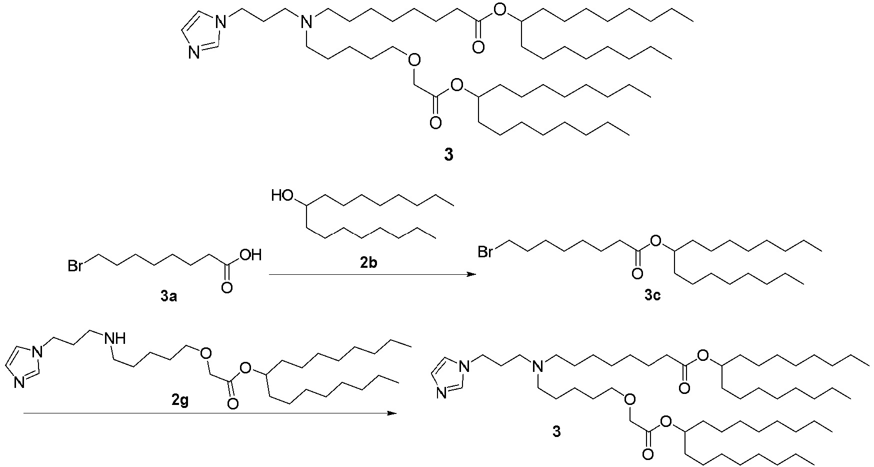

### Step 1: Preparation of compound 3c

Compound 2b (1.92 g, 7.47 mmol), compound 3a (2 g, 8.96 mmol, purchased from Accela ChemBio), and DMAP (912.64 mg, 7.47 mmol) were dissolved in DCM (20 mL), and the solution was stirred at 0 °C. After EDCI (2.15 g, 11.21 mmol) was added, the mixture was naturally warmed to room temperature and left to react for 16 h. 2 M HCl (10 mL) was added, and the aqueous phase was extracted with dichloromethane (20 mL × 2). The organic phases were combined, dried over anhydrous sodium sulfate, then filtered, and concentrated to dryness, and the residue was purified by column chromatography (PE:EA = 0-10%) to give compound 3c (2.78 g, yield: 80.63%).

### Step 2: Preparation of compound 3

Compound 2g (0.1 g, 196.93 µmol) and compound 3c (82.63 mg, 179.03 µmol) were dissolved in acetonitrile (1 mL) and cyclopentyl methyl ether (1 mL). After potassium iodide (32.69 mg, 196.93 µmol) and potassium carbonate (98.97 mg, 716.12 µmol) were added, the mixture was heated to 86 °C and left to react for 16 h. The reaction mixture was cooled to room temperature and then filtered. After the filter cake was washed with acetonitrile, the filtrate was concentrated to dryness, and the residue was purified by reversed-phase C4 column chromatography (H₂O:ACN = 0-100%) to give compound 3 (32.10 mg, yield: 20.18%).

¹H NMR (400 MHz, CDCl₃): δ 7.47 (s, 1H), 7.05 (s, 1H), 6.91 (s, 1H), 5.00-4.91 (m, 1H), 4.90-4.82 (m, 1H), 4.04 (s, 2H), 3.99 (t, J=6.8 Hz, 2H), 3.52 (t, J=6.4 Hz, 2H), 2.42-2.31 (m, 6H), 2.28 (t, J=7.2 Hz, 2H), 1.93-1.81 (m, 2H), 1.68-1.60 (m, 4H), 1.56-1.46 (m, 8H), 1.45-1.34 (m, 6H), 1.33-1.18 (m, 54H), 0.91-0.80 (m, 12H).

### Comparative Example 1

Obtained by referring to the method in WO2021113777.

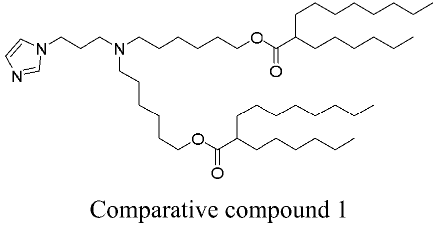

### Comparative Example 2

Obtained by referring to the method in WO2021178396.

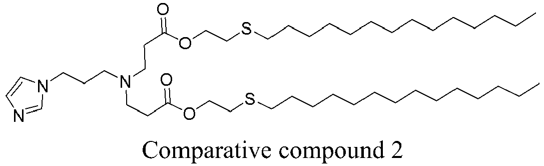

### Comparative Example 3

Obtained by referring to the method in WO2023125738.

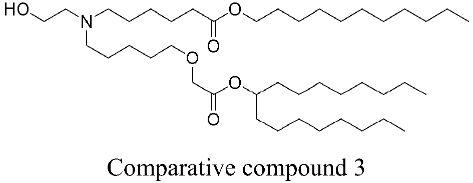

### Example 5: Preparation of Lipid Nanoparticle Compositions

### 1.1. Preparation method

Compounds 1, 2, and 3 and comparative compounds 1, 2, and 3 were each dissolved in an ethanol solution, and each of the resulting solutions was mixed with solutions of DSPC, cholesterol, and DMG-PEG in ethanol in a molar percent (mol %) ratio of 50:10:38.5:1.5 to prepare an ethanol lipid solution. mRNA (luciferase, GenBank: MN728548.1) was dissolved in a 50 mM acetic acid buffer (pH 5) to prepare an aqueous mRNA solution (0.2 mg/mL). Each of the ethanol lipid solutions was mixed with the aqueous mRNA solution by microfluidics, with the volume ratio of the ethanol phase to the aqueous phase being 1:3 and the weight ratio of total lipids to mRNA being about 20:1, to prepare liposomes. Ethanol was removed by dialysis against a 20 mM Tris solution (pH 7.5). Finally, each solution was exchanged into a 20 mM Tris, 8% sucrose solution, and the resulting solutions were cryopreserved to obtain lipid nanoparticle compositions in which mRNA was encapsulated.

### 1.2. Characterization of lipid nanoparticle compositions

The size and polydispersity index (PDI) of the lipid nanoparticles were determined using a Malvern Zetasizer Pro in a 173° backscatter detection mode and dynamic light scattering.

The liposome encapsulation efficiency was determined using a Quant-iT RiboGreen RNA assay kit.

The results are shown in Table 1 below.

**Table 1**

| No. | Size (nm) | PDI | Encapsulation efficiency (%) |
|---|---|---|---|
| Compound 1 | 79.4 | 0.07 | 95.5 |
| Compound 2 | 78.1 | 0.10 | 99.4 |
| Compound 3 | 84.2 | 0.17 | 98.8 |
| Comparative compound 1 | 69.7 | 0.19 | 97.4 |
| Comparative compound 2 | 95.6 | 0.16 | 91.5 |
| Comparative compound 3 | 95.5 | 0.12 | 96.3 |

### Test Example 1: Comparison of Delivery Efficiency of Lipid Nanoparticle Compositions in Immune Cells and Non-Immune Cells

Non-immune cells such as A549 cells (human non-small cell lung cancer cells) and immune cells such as Jurkat cells (human T cells) (from ATCC) were cultured in 96-well plates, respectively. The lipid nanoparticle compositions in which the luciferase mRNA (luciferase, GenBank: MN728548.1) was encapsulated were added to the cell well plates. After 24 h of incubation, the luciferase substrate (D-luciferin potassium salt, Cat. No. abs42075819) was added, and the fluorescence intensity was measured using a microplate reader. The fluorescence intensity indicates the level of luciferase protein expression, i.e., the delivery efficiency of the lipid nanoparticles corresponding to the compounds on different cells. The test for each group was repeated at least 3 times, and the mean was calculated. ns indicates that there is no statistically significant difference, i.e., statistical P > 0.5. ** indicates that there is a statistically significant difference, i.e., statistical P < 0.01. *** indicates that there is a statistically significant difference, i.e., P < 0.001.

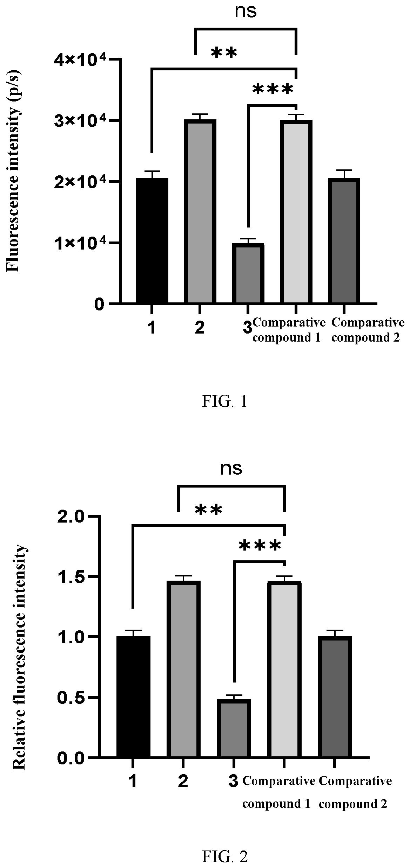

Conclusion: As shown in Tables 2 and 3 and FIGs. 1 and 2, in non-immune cells, the delivery efficiency of the lipid nanoparticle compositions corresponding to compounds 1-3 was not significantly different from that of the lipid nanoparticle compositions corresponding to comparative compounds 1 and 2, or was significantly lower than that of the lipid nanoparticle compositions corresponding to comparative compounds 1 and 2. The relative fluorescence intensity of each group was calculated with the fluorescence intensity of the lipid nanoparticle composition corresponding to comparative compound 2 taken as a unit 1. The relative fluorescence intensities of the lipid nanoparticle compositions corresponding to compounds 1-3 were about 1, indicating that the expression levels of the lipid nanoparticles corresponding to compounds 1-3 on non-immune cells were comparable to or lower than that of the lipid nanoparticle composition corresponding to the comparative compound.

The fluorescence intensities in immune cells to which the lipid nanoparticle compositions corresponding to compounds 1-3 delivered were significantly higher than those of the lipid nanoparticle compositions corresponding to comparative compounds 1 and 2. The fluorescence intensities of the lipid nanoparticle compositions corresponding to compounds 1, 2, and 3 were 9 times, 13 times, and 6 times, respectively, that of the lipid nanoparticle composition corresponding to comparative compound 2.

It can be seen that compared to the lipid nanoparticle compositions corresponding to comparative compounds 1-2, the lipid nanoparticle compositions corresponding to compounds 1-3 exhibited comparable or significantly reduced protein expression in non-immune cells and significantly increased protein expression in immune cells, indicating better immune cell-targeting capability and higher delivery efficiency.

**Table 2. Determination of non-immune cell transfection efficiency**

| | Mean fluorescence intensity (p/s) | Relative fluorescence intensity |
|---|---|---|
| Compound 1 | 2.06E+04 | 1.00 |
| Compound 2 | 3.02E+04 | 1.46 |
| Compound 3 | 9.92E+03 | 0.48 |
| Comparative compound 1 | 3.00E+04 | 1.46 |
| Comparative compound 2 | 2.06E+04 | 1.00 |

**Table 3. Determination of immune cell transfection efficiency**

| | Mean fluorescence intensity (p/s) | Relative fluorescence intensity |
|---|---|---|
| Compound 1 | 2.59E+04 | 9.33 |
| Compound 2 | 3.72E+04 | 13.38 |
| Compound 3 | 1.79E+04 | 6.43 |
| Comparative compound 1 | 8.55E+03 | 3.07 |
| Comparative compound 2 | 2.78E+03 | 1.00 |

### Test Example 2: Assessment of In Vivo mRNA Delivery Efficiency of Tail Vein Injection of Lipid Nanoparticle Compositions in Immune Cells

Lipid nanoparticle compositions in which a Cre-expressing recombinant mRNA (GenBank: JQ033384.1) was encapsulated (prepared according to the preparation method in Example 5) were administered to male CAG-LSL-tdTomato model mice aged 6-8 weeks (Shanghai Model Organisms Center, Inc.) by tail vein injection at a dose of 0.5 mg/kg. If the Cre mRNA was delivered into cells in the mice, it would edit the genes of the mice to enable the expression of the tdTomato fluorescent protein. Therefore, the *in vivo* delivery efficiency of the lipid nanoparticle compositions in different cells can be determined by detecting fluorescence signals of the tdTomato protein and analyzing the proportion of positive cells. Forty-eight hours after administration, the mice were dissected, and their spleens were collected and ground. After lysis of red blood cells, single-cell suspensions were prepared, with cells resuspended in a PBS buffer (pH 7.4). Different immune cells were labeled with fluorescently labeled antibodies (BioLegend), respectively. The cells were divided into populations using a flow cytometer, and the proportion of positive cells (with tdTomato fluorescence signals) in a corresponding cell population was determined, reflecting the delivery efficiency of the nanoparticle lipid compositions in the corresponding immune cells. The blank control group was injected with an equal volume of PBS.

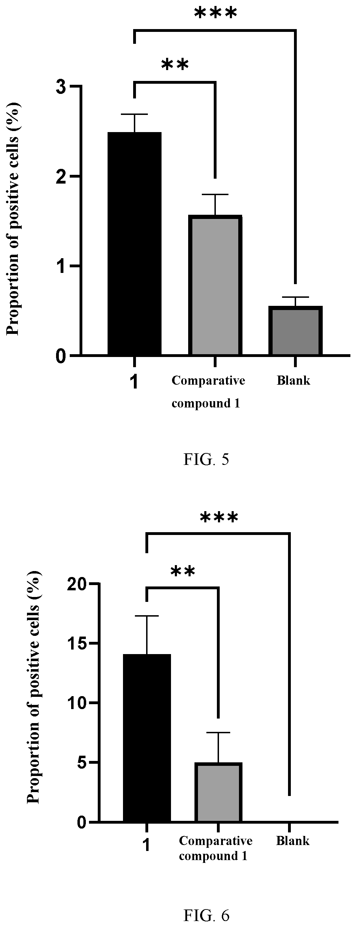

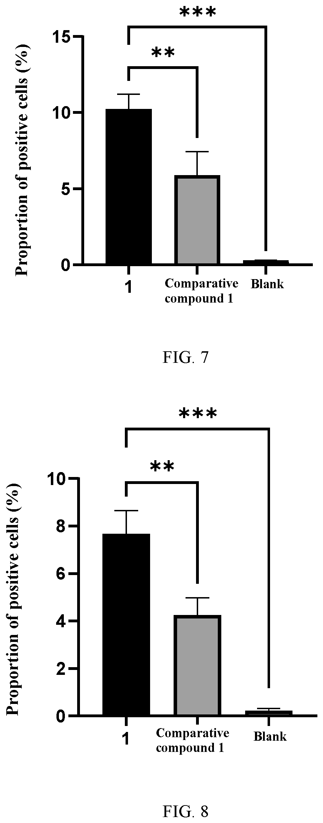

For the lipid nanoparticle composition corresponding to each compound, at least 3 biological replicates were set, and the mean percentage was calculated. The data are shown in Table 4 and FIGs. 5 to 9.

With the lipid nanoparticle composition corresponding to comparative compound 1 and the blank control group as controls, the *in vivo* mRNA delivery efficiency of the tail vein injection of the lipid nanoparticle composition corresponding to compound 1 into mice in immune cells was determined.

**Table 4. In vivo delivery efficiency in immune cells**

| Immune cells | Proportion of positive cells (%) | | |
|---|---|---|---|
| | Compound 1 | Comparative compound 1 | Blank control |
| T cells | 2.5 | 1.6 | 0.6 |
| NK cells | 14.1 | 5.0 | 0.0 |
| Macrophages | 10.2 | 5.9 | 0.3 |
| Dendritic cells | 7.7 | 4.2 | 0.2 |
| Monocytes | 16.8 | 12.3 | 1.3 |

Conclusion: According to the proportions of fluorescent positive cells shown in Table 4 and FIGs. 5 to 9, the lipid nanoparticle composition corresponding to compound 1 could effectively deliver mRNA to T cells, NK cells, macrophages, dendritic cells, and monocytes, and the proportion of positive cells corresponding to compound 1 was significantly increased compared to the blank control group and the lipid nanoparticle composition corresponding to comparative compound 1. This indicates that the lipid nanoparticle composition corresponding to compound 1 can achieve efficient transfection of immune cells *in vivo.*

### Test Example 3: Assessment of Expression Levels of mRNA Delivered by Tail Vein Injection of Lipid Nanoparticle Compositions in Tissues

Lipid nanoparticle compositions in which a luciferase-expressing mRNA (luciferase, GenBank: MN728548.1) was encapsulated (prepared according to the preparation method in Example 5) were administered to female Balb/c mice aged 6-8 weeks by tail vein injection at a dose of 0.5 mg/kg. Six hours after administration, the luciferase substrate (D-luciferin potassium salt, Cat. No. abs42075819) was injected intraperitoneally into each mouse. The mice were dissected, their livers and spleens were collected and photographed using an IVIS small-animal optical *in vivo* imaging system (PerkinElmer), and the fluorescence intensity of each tissue was measured. For the lipid nanoparticle composition corresponding to each compound, at least 3 biological replicates were set, and the mean fluorescence intensity was calculated. The fluorescence intensity indicates the level of luciferase protein expression. The relative fluorescence intensities, i.e., relative protein expression levels, of compounds 1, 2, and 3 and comparative compounds 1 and 3 were calculated with the fluorescence intensity of the lipid nanoparticle composition corresponding to comparative compound 1 taken as a unit 1. For example, the ratio of the tissue fluorescence intensity of compound 1 to the tissue fluorescence intensity of comparative compound 1 is the relative protein expression level of compound 1.

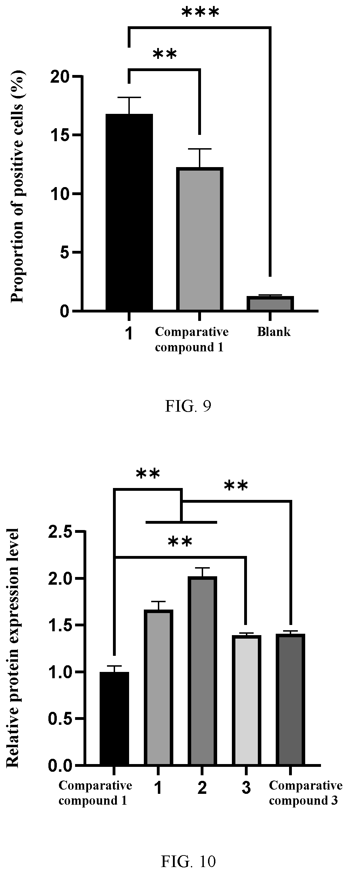

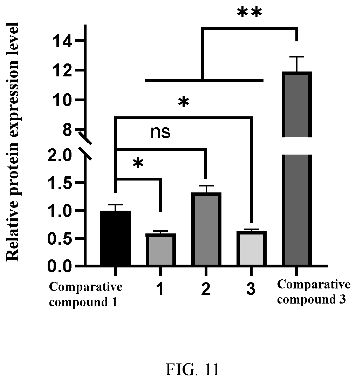

As shown in Table 5 and FIGs. 10 and 11, the relative protein expression levels were calculated using the measured fluorescence intensities corresponding to the spleens and livers of the mice that were photographed using an IVIS small-animal optical *in vivo* imaging system. They measured how efficiently the lipid nanoparticle compositions delivered mRNA in cells of different tissues. In FIGs. 10 and 11, * denotes statistical P < 0.05, ** denotes statistical P < 0.01, and *** denotes statistical P < 0.001, and they indicate that there are statistically significant differences between the groups; ns denotes statistical P ≥ 0.05, which indicates that there is no statistically significant difference between the groups.

**Table 5**

| No. | Relative protein expression level | |
|---|---|---|
| | Spleen | Liver |
| Compound 1 | 1.7 | 0.6 |
| Compound 2 | 2.0 | 1.4 |
| Compound 3 | 1.4 | 0.6 |
| Comparative compound 1 | 1.0 | 1.0 |
| Comparative compound 3 | 1.4 | 12.0 |

Conclusion: The spleen mainly consists of immune cells. The protein expression levels of the lipid nanoparticle compositions corresponding to compounds 1, 2, and 3 in the spleen were significantly higher than that of comparative compound 1, and the protein expression levels of the lipid nanoparticle compositions corresponding to compounds 2 and 3 in the spleen were significantly higher than that of comparative compound 3. In the liver, the protein expression levels corresponding to the lipid nanoparticle compositions corresponding to compounds 1 and 3 were significantly lower than that of comparative compound 1, and the protein expression levels corresponding to the lipid nanoparticle compositions corresponding to compounds 1, 2, and 3 were significantly lower than that of comparative compound 3. This indicates that compounds 1, 2, and 3 have better immune cell delivery abilities, enabling more expression in the spleen while reducing expression in non-immune cells, for example, in the liver.

## Claims

1. A compound represented by formula I or a salt thereof, 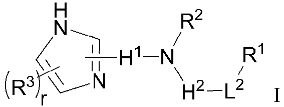 wherein:
H¹ is selected from the group consisting of C₁₋₆ alkylene, C₂₋₆ alkenylene, and C₁₋₆ heteroalkylene;
H² is selected from the group consisting of C₁₋₁₂ heteroalkylene;
R¹ is selected from the group consisting of C₁₋₂₄ alkyl, C₁₋₂₄ heteroalkyl, C₂₋₂₄ alkenyl, and C₂₋₂₄ heteroalkenyl;
R² is selected from the group consisting of C₁₋₂₄ alkyl, C₂₋₂₄ alkenyl, and -R^{b}-L¹-R^{c};
L¹ and L² are each independently selected from the group consisting of -C(O)O-, -OC(O)-, -C(O)-, -OC(O)O-, -O-, -S(O)_{q}-, -S-S-, -C(O)S-, -SC(O)-, -NR^{a}C(O)-, -C(O)NR^{a}-, -NR^{a}C(O)NR^{a}-, -NR^{a}C(O)O-, -OC(O)NR^{a}-, -P(O)(OR^{a})-, and a bond, wherein R^{a} is selected from the group consisting of hydrogen and C₁₋₆ alkyl, and q is selected from the group consisting of 0, 1, and 2;
R^{b} is selected from the group consisting of C₁₋₁₂ alkylene;
R^{c} is selected from the group consisting of C₁₋₂₄ alkyl, C₁₋₂₄ heteroalkyl, C₂₋₂₄ alkenyl, and C₂₋₂₄ heteroalkenyl;
each R³ is independently selected from the group consisting of halogen, amino, hydroxy, C₁₋₆ alkyl, C₁₋₆ alkylamino, C₁₋₆ alkoxy, C₃₋₇ cycloalkyl, and C₃₋₇ heterocycloalkyl;
r is selected from the group consisting of integers between 0 and 3, such as 1 or 2.

2. The compound represented by formula I or the salt thereof according to claim 1, wherein H² is selected from the group consisting of C₁₋₁₂ heteroalkylene, preferably C₂₋₉ heteroalkylene.

3. The compound represented by formula I or the salt thereof according to claim 1 or 2, wherein R¹ is selected from the group consisting of C₁₋₂₄ alkyl, preferably C₄-₁₈ alkyl; or R¹ is selected from the group consisting of C₂₋₂₄ alkenyl, preferably C₄-₁₈ alkenyl.

4. The compound represented by formula I or the salt thereof according to any one of claims 1 to 3, wherein R² is selected from the group consisting of -R^{b}-L²-R^{c}, wherein R^{b}, L², or R^{c} is as defined in claim 1.

5. The compound represented by formula I or the salt thereof according to any one of claims 1 to 4, being a compound represented by formula II or a salt thereof, 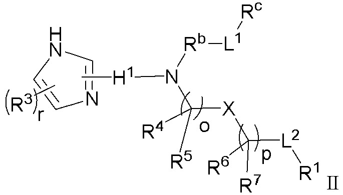 wherein X is -N(R^{d})- or -O-; o is selected from the group consisting of integers between 1 and 11, p is selected from the group consisting of integers between 1 and 5, and o + p is not greater than 12; R^{d} is selected from the group consisting of hydrogen and C₁₋₆ alkyl; R⁴, R⁵, R⁶, and R⁷ are each independently selected from the group consisting of hydrogen, halogen, hydroxy, C₁₋₆ alkyl, and C₁₋₆ alkoxy; L¹, L², H¹, R¹, R³, R^{b}, and R^{c} are as defined in claim 1.

6. The compound represented by formula I or the salt thereof according to claim 5, wherein p is selected from the group consisting of 1 and 2.

7. The compound represented by formula I or the salt thereof according to claim 5, wherein o is selected from the group consisting of integers between 4 and 7, such as 5 or 6.

8. The compound represented by formula I or the salt thereof according to any one of claims 1 to 7, wherein H¹ is selected from the group consisting of C₁₋₆ alkylene, preferably C₃₋₆ alkylene, and more preferably linear C₃₋₆ alkylene.

9. The compound represented by formula I or the salt thereof according to any one of claims 1 to 8, wherein each R³ is independently selected from the group consisting of C₁₋₆ alkyl and C₁₋₆ alkylamino.

10. The compound represented by formula I or the salt thereof according to any one of claims 1 to 8, wherein r = 0.

11. The compound represented by formula I or the salt thereof according to any one of claims 1 to 10, wherein L¹ and L² are each independently selected from the group consisting of -C(O)O-, -OC(O)-, and a bond, preferably -C(O)O-.

12. The compound represented by formula I or the salt thereof according to any one of claims 1 to 11, being a compound represented by formula III-a or formula III-b or a salt thereof: 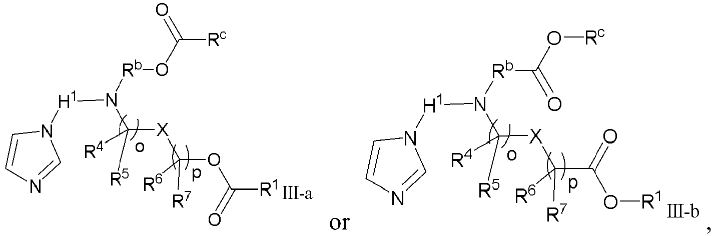 wherein X, H¹, R¹, R⁴-R⁷, R^{b}, R^{c}, o, and p are as defined in claim 5.

13. The compound represented by formula I or the salt thereof according to any one of claims 1 to 12, wherein R^{c} is selected from the group consisting of C₁₋₂₄ alkyl, preferably branched C₁₁₋₁₈ alkyl.

14. The compound represented by formula I or the salt thereof according to any one of claims 1 to 13, wherein R^{b} is selected from the group consisting of C₇₋₉ alkylene.

15. The compound represented by formula I or the salt thereof according to any one of claims 1 to 14, wherein R¹ or R^{c} is selected from the group consisting of: 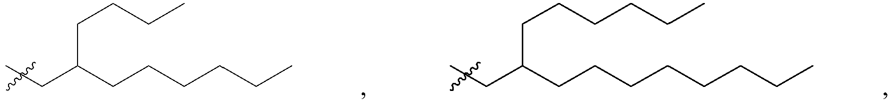 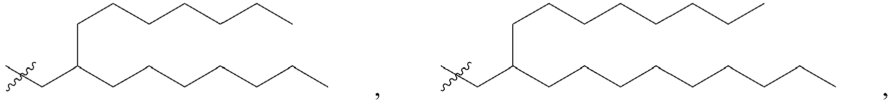 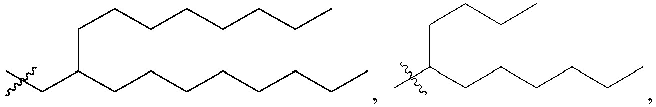 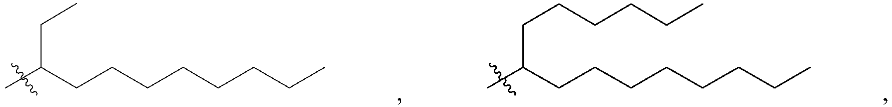 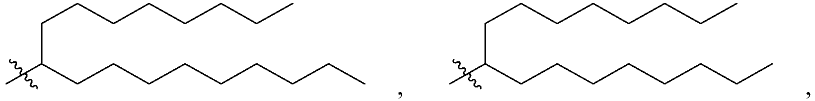 and

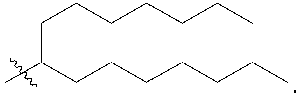

16. The compound represented by formula I or the salt thereof according to any one of claims 1 to 15, wherein the compound is selected from the group consisting of: 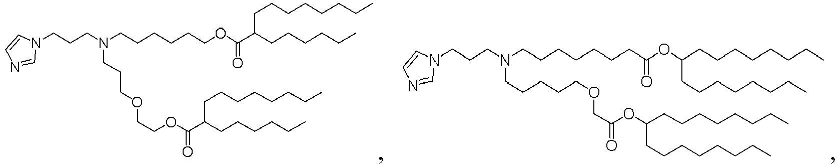 and

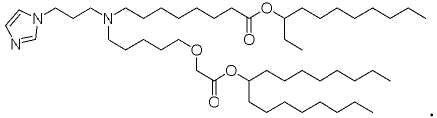

17. An isotopically substituted form of the compound represented by formula I according to any one of claims 1 to 16, wherein preferably, the isotopically substituted form is a deuterium-substituted form.

18. A lipid particle, comprising the compound represented by formula I or the salt thereof according to any one of claims 1 to 16 or the isotopically substituted form according to claim 17, and an active agent, wherein the active agent is preferably a polynucleotide or a nucleic acid, e.g., a DNA, an siRNA, or an mRNA.

19. A pharmaceutical composition, comprising the lipid particle according to claim 18 and a pharmaceutically acceptable excipient.

20. Use of the compound represented by formula I or the salt thereof according to any one of claims 1 to 16, or the isotopically substituted form according to claim 17, or the lipid particle according to claim 18, or the pharmaceutical composition according to claim 19 in the manufacture of a medicament for preventing and/or treating cancer, infectious diseases, autoimmune diseases, neurodegenerative diseases, and inflammation.
